# EUROPEAN PATENT APPLICATION

(11) **EP 3 862 004 A1**
(43) Date of publication of application: **11.08.2021**
(21) Application number: 19869776.5
(22) Date of filing: 27.09.2019
(51) Int. Cl.: A61K 31/7076, A61P 1/16, A61P 31/20, A61P 35/00

(54) **ANTI-HEPATITIS B VIRUS AGENT**

(30) Priority: 04.10.2018 JP 2018189216; 17.04.2019 JP 2019078616
(71) Applicant: DAIKIN INDUSTRIES, LTD., Osaka 530-8323 (JP); Kagoshima University, Kagoshima-shi, Kagoshima 890-8580 (JP)
(72) Inventor: HAMADA, Tomohito, Osaka-shi, Osaka 530-8323 (JP); KISHIKAWA, Yosuke, Osaka-shi, Osaka 530-8323 (JP); INOO, Kanako, Osaka-shi, Osaka 530-8323 (JP); IKEDA, Masanori, Kagoshima-shi, Kagoshima 890-8580 (JP); TAKEDA, Midori, Kagoshima-shi, Kagoshima 890-8580 (JP)
(74) Representative: Hoffmann Eitle
(86) International application number: PCT/JP2019/038246
(87) International publication number: WO 2020/071283

(57) **Abstract**

The present disclosure provides an anti-hepatitis B virus agent and a prophylactic or therapeutic agent for hepatitis B virus-related diseases, each comprising a nucleic acid analogue as an active ingredient.

The problem can be solved by 2'-deoxy-2'-fluoro-β-2-fluoro-D-adenosine or its prodrug, or a pharmaceutically acceptable salt thereof, or a solvate thereof.

## Description

### Technical Field

The present disclosure relates to an anti-hepatitis B virus agent, and a prophylactic or therapeutic agent for a hepatitis B virus-related disease, each comprising a nucleic acid analogue as an active ingredient.

### Background Art

As an active ingredient of an anti-hepatoma virus agent and a prophylactic or therapeutic agent for a hepatoma virus-related disease, a nucleic acid analog represented by the following formula: 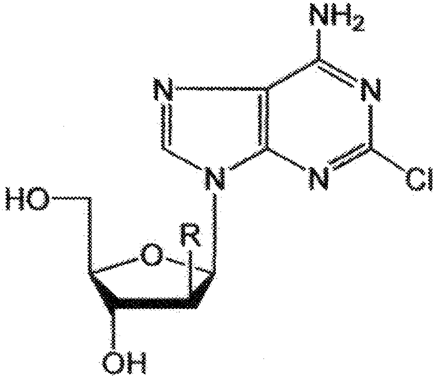 wherein R is fluorine or hydrogen
is known (Patent Literature 1).

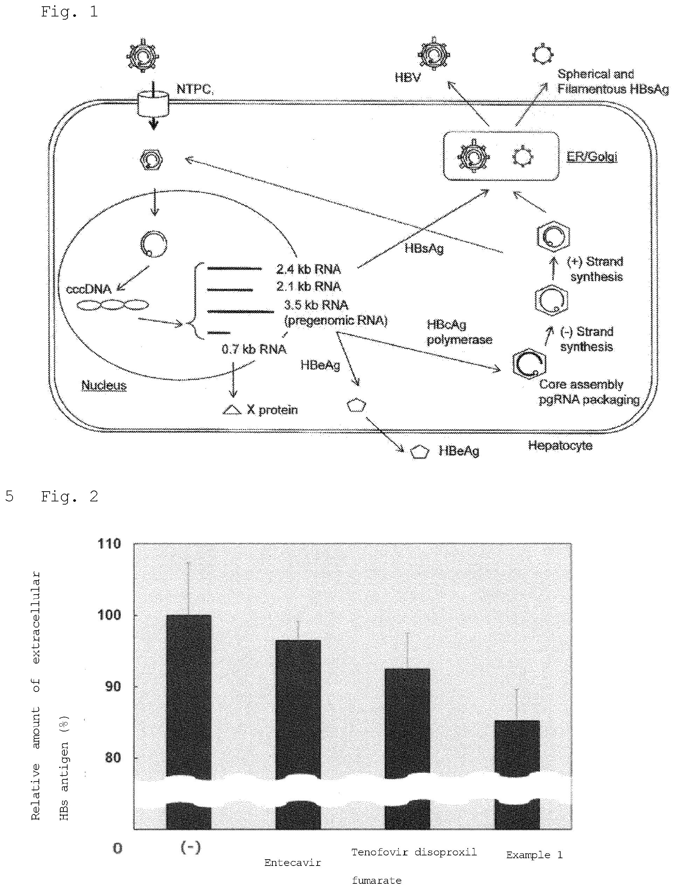

As hepatitis viruses, hepatitis B virus (HBV) and hepatitis C virus (HCV) are known. Of these, the life cycle of HBV is shown in Fig. 1. HBV is an incomplete double-stranded DNA virus belonging to the genus *Orthohepadnavirus* of the family Hepadnaviridae. Once HBV invades cells, it forms complete double-stranded DNA in the nucleus, which results in covalently closed circular DNA (cccDNA). 3.5-, 2.4-, 2.1-, and 0.7-kb mRNAs are transcribed using DNA as a template, and translated into a polymerase, HBcAg (Core), HBsAg, and an X protein, respectively. 3.5-kb pregenomic RNA (pgRNA) is packaged together with Core and the polymerase. After pgRNA is reverse-transcribed into DNA, viral particles are released extracellularly. Although HBV is not a retrovirus, as it has reverse transcription activity to polymerase, a reverse transcriptase inhibitor against HIV-1 is used for treatment of HBV.

### Citation List

### Patent Literature

PTL 1: WO2017/155082

### Summary of Invention

### Technical Problem

The present disclosure aims to provide an anti-hepatitis B virus agent, and a prophylactic or therapeutic agent for a hepatitis B virus-related disease, each comprising a nucleic acid analogue as an active ingredient.

### Solution to Problem

The present disclosure includes the following embodiments.
Item 1. An anti-hepatitis B virus agent comprising, as an active ingredient, 2'-deoxy-2'-fluoro-β-2-fluoro-D-adenosine or its prodrug, or a pharmaceutically acceptable salt thereof, or a solvate thereof.
Item 2. A prophylactic or therapeutic agent for a hepatitis B virus-related disease, comprising, as an active ingredient, 2'-deoxy-2'-fluoro-β-2-fluoro-D-adenosine or its prodrug, or a pharmaceutically acceptable salt thereof, or a solvate thereof.
Item 3. The prophylactic or therapeutic agent according to Item 2, wherein the hepatitis B virus-related disease is one or more diseases selected from the group consisting of hepatitis B, type B liver cirrhosis, and type B liver cancer.

The present disclosure further includes the following embodiments.
- 2'-Deoxy-2'-fluoro-β-2-fluoro-D-adenosine or its prodrug, or a pharmaceutically acceptable salt thereof, or a solvate thereof, for use as a medicament for preventing or treating a hepatitis B virus-related disease.
- Use of 2'-deoxy-2'-fluoro-β-2-fluoro-D-adenosine or its prodrug thereof, or a pharmaceutically acceptable salt thereof, or a solvate thereof, for the manufacture of a medicament for preventing or treating a hepatitis B virus-related disease.

### Advantageous Effects of Invention

The present disclosure provides an anti-hepatitis B virus agent, and a prophylactic or therapeutic agent for a hepatitis B virus-related disease, each comprising a nucleic acid analogue as an active ingredient.

### Brief Description of Drawings

Fig. 1 illustrates the life cycle of HBV.
Fig. 2 is a graph showing a comparison of the relative amount of extracellular HBs antigen between the compound of Example 1 and a known anti-hepatitis B virus agent.

### Description of Embodiments

The above summary of the present disclosure is not intended to describe each disclosed embodiment or every implementation of the present disclosure.

The description of the present disclosure that follows more specifically exemplifies illustrative embodiments.

In several places throughout the present disclosure, guidance is provided through lists of examples, and these examples can be used in various combinations.

In each instance, the described list serves only as a representative group, and should not be interpreted as an exclusive list.

All of the publications, patents, and patent applications cited herein are incorporated herein by reference in their entirety.

### Terms

Unless otherwise specified, the symbols and abbreviations herein can be understood in the context of the present specification in the meanings commonly used in the art to which the present disclosure belongs.

The term "contains" and "comprises" as used herein is intended to include "consisting essentially of" and "consisting of."

Unless otherwise specified, the steps, treatments, or operations described herein can be performed at room temperature.

The room temperature referred to herein can mean a temperature in the range of 10 to 40°C.

The notation "Cₙ₋ₘ" (where n and m are each a number) used herein means that the number of carbon atoms is n or more and m or less, as is usually understood by persons skilled in the art.

Examples of the halogen atom in the present specification include fluorine, chlorine, bromine, and iodine, unless otherwise specified.

Examples of the alkyl group in the present specification include linear or branched C₁₋₂₀ alkyl (e.g., methyl, ethyl, propyl (n-propyl, and isopropyl), butyl (n-butyl, s-butyl, i-butyl, and t-butyl), pentyl, hexyl, heptyl, octyl, 2-ethylhexyl, nonyl, decyl, undecyl, dodecyl, tridecyl, tetradecyl, pentadecyl, hexadecyl, heptadecyl, and octadecyl).

Examples of the cycloalkyl group in the present specification include C₃₋₁₀ cycloalkyl (e.g., cyclopentyl and cyclohexyl).

Examples of the aryl group in the present specification include C₆₋₁₀ aryl (e.g., phenyl and naphthyl) .

Examples of the aralkyl group in the present specification include C₆₋₁₀ aryl-linear or branched C₁₋₁₀ alkyl (e.g., benzyl and phenethyl).

Examples of the alkoxy group in the present specification include linear or branched C₁₋₁₀ alkyloxy (e.g., methoxy, ethoxy, and propoxy).

Examples of the alkoxyalkoxy group in the present specification include linear or branched C₁₋₂₀ alkyloxy, linear or branched C₁₋₄ alkyloxy (e.g., methoxymethoxy, methoxyethoxy, and ethoxyethoxy, hexadecyloxypropoxy, and octadecyloxyethoxy).

Examples of the acyloxy group in the present specification include linear or branched C₁₋₁₀ alkylcarbonyloxy (e.g., methylcarbonyloxy, ethylcarbonyloxy, and propylcarbonyloxy) .

Examples of substituents that each of the alkyl, cycloalkyl, aryl, aralkyl, alkoxy, alkoxyalkoxy, acyloxy, and steroid groups may have include halogens and organic groups. Preferable examples thereof include fluorine, chlorine, bromine, alkoxy, alkylcarbonyloxy, alkylcarbonylthio, alkyloxycarbonyl, and alkyldithio. Most preferable examples thereof include halogens, linear or branched C₁₋₂₀ alkyloxy, linear or branched C₁₋₁₀ alkylcarbonyloxy, linear or branched C₁₋₁₀ alkylcarbonylthio, linear or branched C₁₋₁₀ alkyloxycarbonyl, and linear or branched C₁₋₁₀ alkyldithio.

### Anti-hepatitis B virus agent

In the present disclosure, the "anti-hepatitis B virus agent" means an agent that delays or inhibits the growth of hepatitis B virus.

Hepatitis B virus may be a strain that is resistant to known anti-hepatitis B virus agents (e.g., Entecavir, Tenofovir, and Adefovir Pivoxil).

A "strain that is resistant to known anti-hepatitis B virus agents" means a strain that does not exhibit a growth retardation effect or a growth inhibition effect that normal strains exhibit by the anti-hepatitis B virus agent, or a strain that exhibits a lower growth retardation effect or lower growth inhibition effect than that of normal strains by the anti-hepatitis B virus agent.

The anti-hepatitis B virus agent according to one embodiment of the present disclosure is an anti-hepatitis B virus agent comprising, as an active ingredient, 2'-deoxy-2'-fluoro-β-2-fluoro-D-adenosine (also known as (2R,3R,4S,5R)-5-(6-amino-2-fluoro-9H-purin-9-yl)-4-fluoro-2-(hydroxymethyl)tetrahydrofuran-3-ol), or its prodrug, or a pharmaceutically acceptable salt thereof, or a solvate thereof.

### 2'-Deoxy-2'-fluoro-β-2-fluoro-D-adenosine

The method of producing 2'-deoxy-2'-fluoro-β-2-fluoro-D-adenosine is not limited. For example, the method comprises step A of reacting a compound represented by the following formula (I): 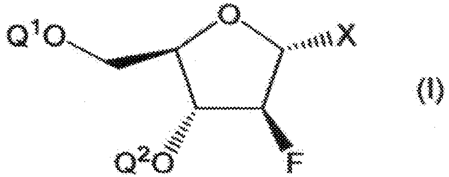 wherein Q¹ and Q² are the same or different, and each represents a protecting group of a hydroxyl group, and X represents bromine or iodine,
with 2-fluoroadenine, and step B of deprotecting the protecting group of the hydroxyl group of the product obtained by the reaction.

### Step A

In Formula (I), Q¹ and Q² are not limited, as long as they are functional groups capable of protecting the hydroxyl group. Examples include ether-type protecting groups (e.g., t-butyl, benzyl, and trityl), acetal-type protecting groups (e.g., tetrahydropyranyl), acyl-type protecting groups (e.g., acetyl and benzoyl), and silyl ether-type protecting groups (e.g., t-butyldimethylsilyl).

The amount of the compound represented by Formula (I) to be used, and the amount of 2-fluoroadenine to be used are not limited as long as the reaction proceeds. The molar ratio of the compound represented by Formula (I) and 2-fluoroadenine can be in the range of 1:1 to 1:5.

The reaction of step A is usually carried out in the presence of a base. The base is preferably a non-nucleophilic base, and examples include metal alkoxides (e.g., sodium t-butoxy and potassium t-butoxy).

The reaction of step A is usually performed in a solvent.

Examples of the solvent include halogen-based solvents (e.g., dichloromethane, chloroform, and dichloroethane); alcohol-based solvents (e.g., ethanol, propanol, butanol, and pentanol); nitrile-based solvents (e.g., acetonitrile); and mixtures of these solvents.

The reaction temperature of step A is not limited as long as the reaction proceeds. It is, for example, in the range of 15 to 80°C.

The reaction time of step A can be set such that the target product is sufficiently obtained, and step A can be continued until the reaction completes.

### Step B

The method and conditions for deprotecting the protecting group of the hydroxy group of the product obtained by the reaction of step A can be selected according to the type of protecting group. For example, a benzoyl group can be deprotected by a reaction with a metal alkoxide (such as sodium methoxide).

The reaction temperature of step B is not limited as long as the reaction proceeds. It is, for example, in the range of 15 to 80°C.

The reaction time of step B can be set such that the target product is sufficiently obtained, and step B can be continued until the reaction completes.

The product obtained by the reaction of steps A and B may be purified by filtration, column chromatography, or the like, as needed.

The method of producing the compound represented by formula (I) is not limited. For example, it is a method comprising the step of reacting a compound represented by the following formula (II): 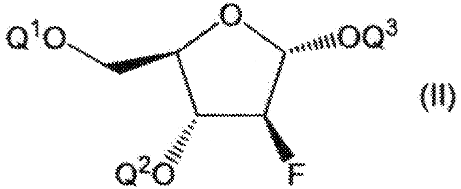 wherein Q¹ and Q² are each as defined above, and Q³ represents a protecting group of a hydroxyl group,
with hydrogen bromide or hydrogen iodide.

The amount of the compound represented by Formula (II) to be used, and the amount of hydrogen bromide or hydrogen iodide to be used are not limited as long as the reaction proceeds. The molar ratio of the compound represented by Formula (II) and hydrogen bromide or hydrogen iodide is, for example, in the range of 1:1 to 1:5.

The reaction of the compound represented by Formula (II) and hydrogen bromide or hydrogen iodide is usually performed in a solvent.

Examples of the solvent include halogen-based solvents (e.g., dichloromethane, chloroform, and dichloroethane); carboxylic acid-based solvents (e.g., acetic acid), and mixtures of these solvents.

The reaction temperature is not limited as long as the reaction proceeds; and is, for example, in the range of 15 to 30°C.

### Prodrug

The prodrug of 2'-deoxy-2'-fluoro-β-2-fluoro-D-adenosine is not limited as long as it can be converted to its active metabolite or 2'-deoxy-2'-fluoro-β-2-fluoro-D-adenosine *in vivo.* Any prodrug that is used as a prodrug of a nucleic acid analogue can be used.

Typical examples of the prodrug include esters and ester amides.

Examples of the esters include phosphoric acid esters. Preferable example thereof include a phosphoric acid monoester represented by the following formula: 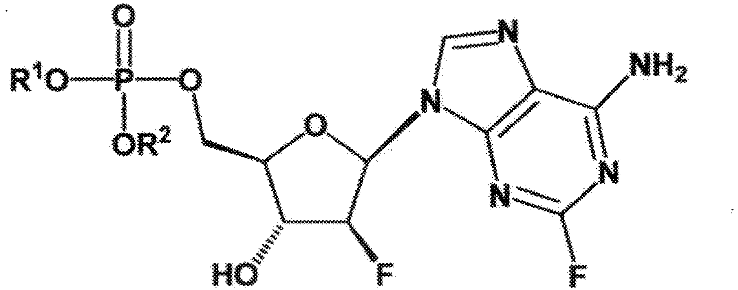 wherein R¹ and R² are the same or different, and each represents a hydrogen atom, an alkyl group optionally having one or more substituents, a cycloalkyl group optionally having one or more substituents, an aryl group optionally having one or more substituents, or an aralkyl group optionally having one or more substituents; or R¹ and R² are bonded each other to form a ring together with phosphorus and oxygen atoms constituting a phosphoric acid ester moiety in the structural formula; and a phosphoric acid di- or tri-ester represented by the following formula: 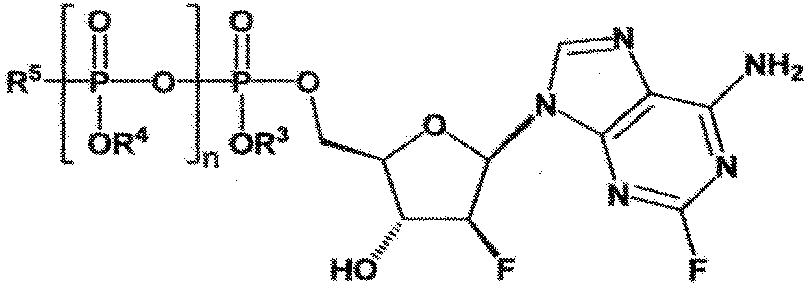 wherein
R³, and R⁴ in each occurrence are the same or different, and each represents a hydrogen atom, an alkyl group optionally having one or more substituents, a cycloalkyl group optionally having one or more substituents, an aryl group optionally having one or more substituents, or an aralkyl group optionally having one or more substituents;
R⁵ represents an alkyl group optionally having one or more substituents, an alkoxy group optionally having one or more substituents, an alkoxyalkoxy group optionally having one or more substituents, an acyloxy group optionally having one or more substituents, or a steroid group optionally having one or more substituents (a group containing cyclopentaphenanthrene or hydrogenated cyclopentaphenanthrene); and
n represents 1 or 2.

R¹ preferably represents an alkyl group optionally having one or more substituents, and more preferably an alkyl group optionally having one or more substituents selected from the group consisting of alkoxy, alkylcarbonyloxy, alkylcarbonylthio, and alkyldithio.

R² preferably represents a hydrogen atom, an alkyl group optionally having one or more substituents, an aryl group optionally having one or more substituents, or an aralkyl group optionally having one or more substituents; and more preferably a hydrogen atom, an alkyl group, an aryl group, or an aralkyl group.

The ring that is formed by binding R¹ and R² each other, together with a phosphorus atom and an oxygen atom constituting a phosphoric acid ester moiety, can be a monocyclic ring or a fused ring.

The number of constituent atoms of the ring is, for example, an integer in the range of 6 to 10.

Examples of the ring include rings represented by the following formulae:

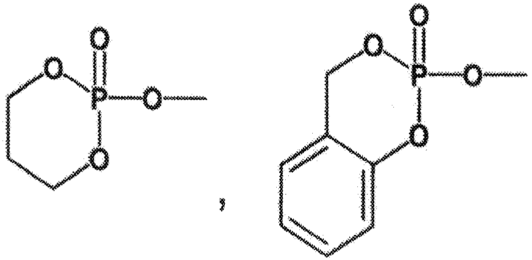

The ring optionally has one or more substituents. Examples of the substituent include halogen, alkyl, cycloalkyl, aryl, and aralkyl.

R³ and R⁴ preferably represent a hydrogen atom, an alkyl group optionally having one or more substituents, an aryl group optionally having one or more substituents, or an aralkyl group optionally having one or more substituents; and even more preferably a hydrogen atom, an alkyl group, an aryl group, or an aralkyl group.

R⁵ preferably represents an alkyl group, an alkoxy group, an alkoxyalkoxy group, an acyloxy group, or a steroid group.

Specific examples of the ester amide include phosphoric acid ester amides. Preferable examples include a compound represented by the following formula: 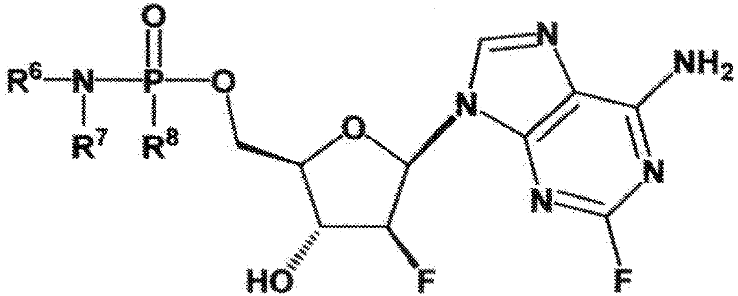 wherein
R⁸ represents -NR^{8a}R^{8b} or -OR^{8c}; and
R⁶, R⁷, R^{8a}, R^{8b}, and R^{8c} are the same or different, and each represents a hydrogen atom, an alkyl group optionally having one or more substituents, a cycloalkyl group optionally having one or more substituents, an aryl group optionally having one or more substituents, or an aralkyl group optionally having one or more substituents.

R⁶ and R^{8a} preferably represent an alkyl group optionally having one or more substituents, and more preferably an alkyl group optionally having a substituent selected from the group consisting of halogen and alkyloxycarbonyl.

R⁷ and R^{8b} preferably represent a hydrogen atom or an alkyl group optionally having one or more substituents, and even more preferably a hydrogen atom or an alkyl group.

R^{8c} preferably represents a hydrogen atom, an alkyl group optionally having one or more substituents, an aryl group optionally having one or more substituents, an aryl group optionally having one or more substituents, an aralkyl group optionally having one or more substituents; and more preferably a hydrogen atom, an alkyl group, an aryl group, or an aralkyl group.

More preferable examples of the prodrug include compounds represented by the following formulae:

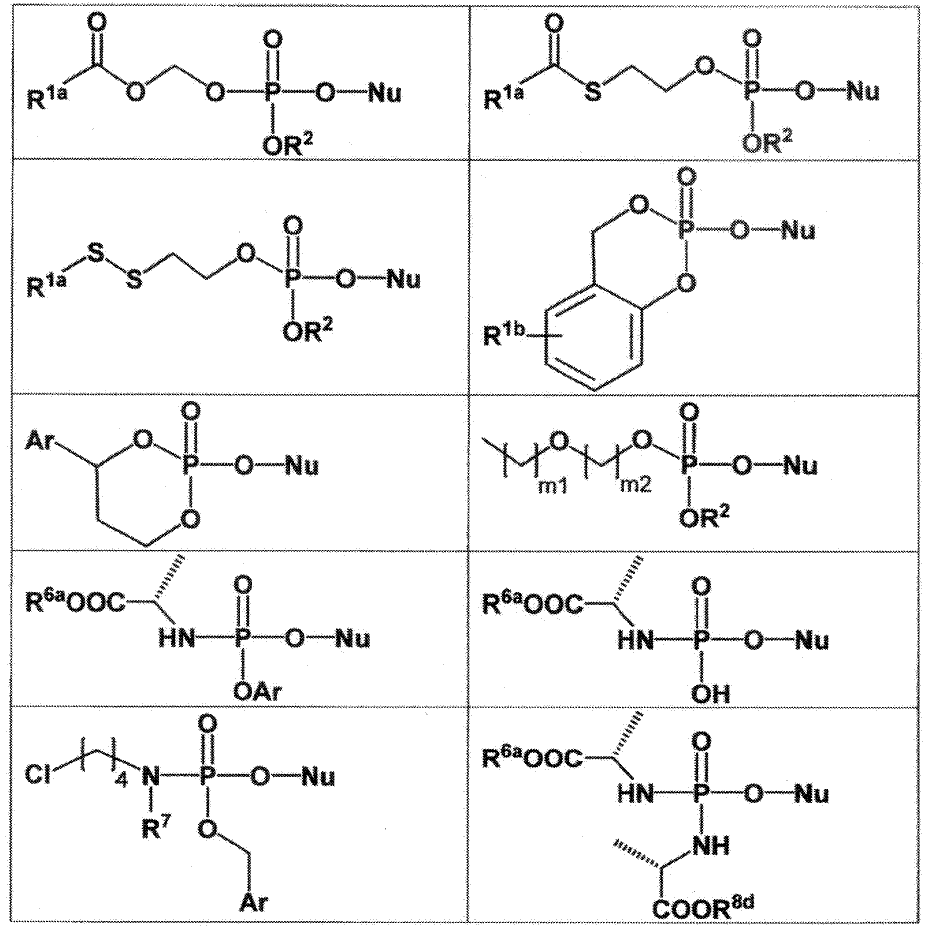

wherein
R^{1a}, R^{6a}, and R^{8d} each represents an alkyl group;
R^{1b} represents halogen or an alkyl group;
R² and R⁷ are as defined above;
Ar represents an aryl group; and
Nu represents a group represented by the following formula: 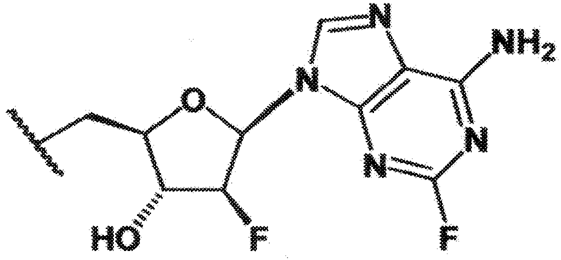 wherein m1 represents an integer in the range of 1 to 18, and m2 represents an integer in the range of 1 to 10.

The prodrug can be produced according to its chemical structure based on technical knowledge with reference to a known method (e.g., a method described in Chemical Reviews 2014, vol. 114, pp. 9154-9218).

### Salt

Examples of the pharmaceutically acceptable salt of 2'-deoxy-2'-fluoro-β-2-fluoro-D-adenosine or its prodrug include
(1) salts with an inorganic acid (e.g., hydrochloric acid, sulfuric acid, phosphoric acid, hydrobromic acid, hydriodic acid, nitric acid, pyrosulfuric acid, and metaphosphoric acid);
(2) salts with an organic acid (e.g., citric acid, benzoic acid, acetic acid, propionic acid, fumaric acid, maleic acid, and sulfonic acid (e.g., methanesulfonic acid, p-toluenesulfonic acid, and naphthalenesulfonic acid)); and
(3) alkali metal salts (e.g., sodium salts and potassium salts).

### Solvate

Examples of the solvate of 2'-deoxy-2'-fluoro-β-2-fluoro-D-adenosine or its prodrug, or a pharmaceutically acceptable salt thereof include hydrates and organic solvates (e.g., methanol solvates, ethanol solvates, and dimethyl sulfoxide solvates).

### Other active ingredients

The anti-hepatitis B virus agent may further contain other active ingredients.

Examples of the "other active ingredients" include other nucleic acid analogues (such as 2'-deoxy-2'-fluoro-nucleoside), and other anti-hepatitis B virus agents.

Two or more anti-hepatitis B virus agents may be used.

2'-Deoxy-2'-fluoro-β-2-fluoro-D-adenosine or its prodrug, or a pharmaceutically acceptable salt thereof, or a solvate thereof may be each formulated into a formulation separated from the "other active ingredient."

2'-Deoxy-2'-fluoro-β-2-fluoro-D-adenosine or its prodrug, or a pharmaceutically acceptable salt thereof, or a solvate thereof may be administered simultaneously, sequentially, or alternately with the "other active ingredient."

### Content of active ingredient

The lower limit of the content of the active ingredient can be set to, for example, 0.001 mass%, preferably 0.01 mass%, and more preferably 0.05 mass%, relative to the total mass of the anti-hepatitis B virus agent in terms of activity. The upper limit of the content of the active ingredient is not limited, and can be set to, for example, 99.99 mass%, preferably 90 mass%, and more preferably 80 mass%, relative to the total mass of the anti-hepatitis B virus agent. The amount of the active ingredient is in the range in which the lower limit and the upper limit are arbitrary selected. For example, the amount of the active ingredient is in the range of 0.001 to 99.99 mass%, preferably 0.01 to 90 mass%, and more preferably 0.05 to 80 mass%.

### Additive

The anti-hepatitis B virus agent may include a pharmaceutically acceptable additive.

Examples of the form of the anti-hepatitis B virus agent include solid formulations (e.g., granules, sprays, tablets, capsules, and dry syrups), semi-solid formulations (e.g., creams, ointments, and gels), and liquid formulations (e.g., solutions and suspensions).

The solid formulation can be produced, for example, by mixing an active ingredient and an additive (e.g., an excipient, binder, disintegrant, lubricant, and colorant); and if necessary, by granulation, particle size regulation, compression, and/or coating.

Examples of the excipient include lactose, lactose hydrate, sucrose, mannitol, sorbitol, crystalline cellulose, starch (e.g., cornstarch), hydrous silicon dioxide, and combinations thereof.

Examples of the binder include agar, gum arabic, hyaluronic acid, hydroxyethyl cellulose, hydroxypropyl cellulose, hydroxypropyl methyl cellulose, and combinations thereof.

Examples of the disintegrant include alginic acid, carboxymethyl cellulose (carmellose), croscarmellose sodium, low substituted hydroxypropyl cellulose, polyvinylpyrrolidone (povidone), crospovidone, and combinations thereof.

Examples of the lubricant include stearic acid, magnesium stearate, calcium stearate, talc, and combinations thereof.

Examples of the colorant include iron trioxide, titanium oxide, and combinations thereof.

The semi-solid formulation can be produced, for example, by mixing an active ingredient, a semi-solid carrier, and optionally other additives.

The liquid formulation can be produced, for example, by mixing an active ingredient, a liquid carrier (e.g., aqueous carrier (e.g., purified water), and oily carrier), and optionally other additives (e.g., an emulsifier, dispersant, suspending agent, buffer, antioxidant, surfactant, osmotic pressure regulator, chelating agent, and antimicrobial agent); and by sterilizing, as necessary.

### Form of administration

The method of administering the anti-hepatitis B virus agent includes oral or non-oral administration (e.g., intravenous, intramuscular, or subcutaneous administration).

The anti-hepatitis B virus agent may be topically administered.

The anti-hepatitis B virus agent may be administered to humans, non-human mammals (e.g., monkeys, sheep, dogs, mice, and rats), and non-mammals.

The number of administrations of the anti-hepatitis B virus agent can be selected according to the age, weight, medical condition, etc. of the subject. The anti-hepatitis B virus agent can be administered, for example, once, twice, or three times a day; once every two days; once every three days; once every four days; once every five days; once every six days; or once a week.

The single dose of the anti-hepatitis B virus agent may range from 0.1 mg to 1000 mg, depending on the target of administration and the frequency of administration.

Preferable examples of the anti-hepatitis B virus agent include orally administered formulations. Examples include a tablet containing an active ingredient, crystalline cellulose, hydroxypropyl methylcellulose, povidone, magnesium stearate, and titanium oxide; and a hard gelatin capsule containing an active ingredient, povidone, and magnesium stearate.

### Prophylactic or therapeutic agent for hepatitis B virus-related diseases

The term "hepatitis B virus-related disease" means a disease that occurred as a result of infection with hepatitis B virus. The hepatitis B virus-related disease can be at least one member selected from the group consisting of hepatitis B (acute hepatitis B, chronic hepatitis B), type B liver cirrhosis, and type B liver cancer.

The prophylactic or therapeutic agent for a hepatitis B virus-related disease according to one embodiment of the present disclosure comprises, as an active ingredient, 2'-deoxy-2'-fluoro-β-2-fluoro-D-adenosine or its prodrug, or a pharmaceutically acceptable salt thereof, or a solvate thereof.

The dosage form and the administration form (e.g., administration route, administration target, administration frequency, and dosage) of other active ingredients and additives that can be contained in the prophylactic or therapeutic agent, and the prophylactic or therapeutic agent can be selected from those mentioned for the anti-hepatitis B virus agent described above.

### Method of delaying or inhibiting growth of hepatitis B virus, or method of preventing or treating hepatitis B virus-related disease

The method of delaying or inhibiting the growth of hepatitis B virus, or the method of preventing or treating a hepatitis B virus-related disease according to one embodiment of the present disclosure includes the step of administering, to a subject, 2'-deoxy-2'-fluoro-β-2-fluoro-D-adenosine or its prodrug, or a pharmaceutically acceptable salt thereof, or a solvate thereof, as necessary.

The administration form (e.g., administration route, administration target, administration frequency, and dosage) can be selected from those described for the anti-hepatitis B virus agent described above.

### Examples

One embodiment of the present disclosure is described in more detail by means of examples; however, the present disclosure is not limited thereto.

### Example 1 (Synthetic Example)

### (1)

2 g of 2-deoxy-2-fluoro-1,3,5-tri-O-benzoyl-α-D-arabinofuranose was dissolved in 20 mL of dichloromethane; and 4 mL of hydrogen bromide acetic acid solution (5.1 mol/L) was added thereto, followed by stirring. After completion of the reaction, a saturated sodium bicarbonate aqueous solution was added to neutralize the unreacted HBr.

Thereafter, separation was conducted; and the solution extracted with dichloromethane was concentrated, thereby obtaining 1.7 g of a crude of 1-bromo-2-deoxy-2-fluoro-3,5-di-O-benzoyl-α-D-arabinofuranose, which was used in the next step.

### (2) Reaction with 2-fluoroadenine

1 g of 2-fluoroadenine was dispersed in a co-solvent of t-amyl alcohol:acetonitrile.

Potassium t-butoxy was added thereto; and the mixture was heated to 50°C, stirred for a while, and dissolved.

1.7 g of a crude of 1-bromo-2-deoxy-2-fluoro-3,5-di-O-benzoyl-α-D-arabinofuranose that had been dissolved in acetonitrile was added thereto dropwise, and subjected to a reaction.

After completion of the reaction, the solid component was diluted with dichloroethane and subjected to filtration. Thereafter, the filtrate was concentrated, and purified by column chromatography.

200 mg of a reaction product (i.e., 2'-deoxy-2'-fluoro-3',5'-di-O-benzoyl-β-2-fluoro-D-adenosine) was obtained.

### (3) Deprotection

100 mg of 2'-deoxy-2'-fluoro-3',5'-di-O-benzoyl-β-2-fluoro-D-adenosine was dissolved in methanol; and 10 mg of sodium methoxide was added thereto, followed by stirring.

After completion of the reaction, the reaction product was purified by preparative TLC, thereby obtaining 60 mg of 2'-deoxy-2'-fluoro-β-2-fluoro-D-adenosine (the following formula). 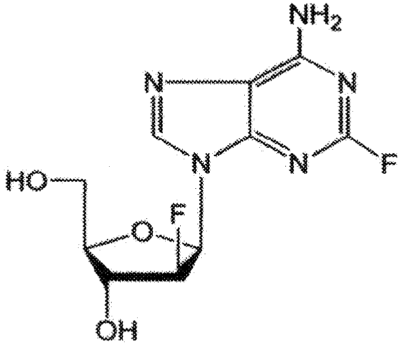 1H NMR (400 MHz, ACETONE-D6) δ 8.14-8.19 (m, 1H), 7.08 (br, 2H), 6.36 (dd, J = 15.3, 4.3 Hz, 1H), 5.14-5.29 (m, 2H), 4.61-4.68 (m, 1H), 4.29 (br, 1H) 3.71-3.99 (m, 3H)

### Comparative Example 1 (Synthetic Example)

In step (2) of Example 1, the same operation was repeated as in Example 1, except that 2,6-dichloropurine was used in place of 2-fluoroadenine to obtain 2,6-dichloro-9-((2R,3S,4R,5R)-3-fluoro-4-hydroxy-5-(hydroxymethyl) tetrahydrofuran-2-yl)-9H-purine (the following formula). 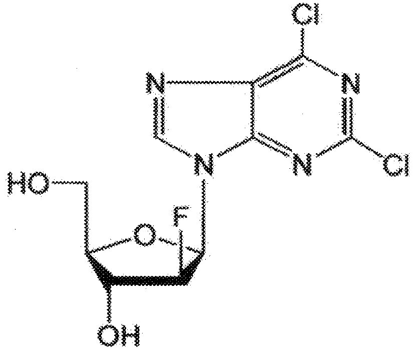 1H NMR (400 MHz, DMSO-D6) δ 8.53 (s, 1H), 6.41 (dd, J = 12.3, 4.6 Hz, 1H), 5.97 (s, 1H), 5.25 (dt, J =52.8, 4.6 Hz, 1H), 5.09 (t, J = 5.5 Hz, 1H), 4.38-4.43 (m, 1H), 3.81-3.91 (m, 1H), 3.58-3.68 (m, 2H)

### Test Example 1 (Anti-HBV Activity)

Anti-HBV activity was evaluated by quantitative PCR for determining the amount of HBV DNA in cells on the seventh day after the addition of the agent, using the HepG2.2.15 cell line of HBV-producing cells obtained by introducing a gene having a length twice that of the HBV genome into the human liver cancer cell line HepG2. For PCR of HBV DNA, a forward primer (HBV-S190F; 5'-GCT CGT GTT ACA GGC GGG-3': SEQ ID NO: 1) and a reverse primer (HBV-S703R; 5'-GAA CCA CTG AAC AAA TGG CAC TAG TA-3': SEQ ID NO: 2) were used. PCR was performed by carrying out a reaction at 95°C for 10 sec, 62°C for 10 sec, and 72°C for 30 sec for 35 cycles.

Specifically, HepG2.2.15 cells were seeded at a concentration of 1 × 10⁵ cells/well. 24 hours later, the compounds of Example 1 and Comparative Example 1 were diluted to have a concentration of 0, 0.49, 0.97, 1.95, 3.90, 7.81, 15.6, 31.3, 62.5, 125, 250, 500, and 1000 nM; and added to the cells. After the cells were cultured for 7 days, each cytoplasmic compartment was collected, and DNA was purified by phenol-chloroform extraction. 20 ng of the purified DNA was used to measure the amount of intracellular HBV DNA by quantitative PCR.

### Test Example 2 (HBs Antigen Level)

The HBs antigen level was measured by measuring the culture supernatant on the fourth day after the addition of the agent by chemiluminescent immunoassay (CLIA) using HepG2.2.15, i.e., HBV-producing cells obtained by introducing a gene having a length twice that of the HBV genome into the human liver cancer cell line HepG2.

### Test Example 3 (Cytotoxicity Test)

HepG2 NTCP-myc cells were seeded at a concentration of 2 x 10⁴ cells/well. 24 hours later, agents were diluted to have a concentration of 0, 0.49, 0.97, 1.95, 3.90, 7.81, 15.6, 31.3, 62.5, 125, 250, 500, and 1000 nM; and added to the cells. The HepG2 NTCP-myc cells were cultured for 7 days. After the culture, 10 µl of Premix WST-1 Cell Proliferation Assay System (TaKaRa) was added, and the cells were cultured at 37°C for 2 hours. Thereafter, absorbance was determined at 450 nm using a microplate reader.

### Results of Test Examples 1 to 3

### 1. Anti-HBV Activity

The EC₅₀ (50% effective concentration) was calculated from a graph showing the relationship between the concentration of the compound and anti-HBV activity.

**Table 1**

| | EC₅₀ (nM) |
|---|---|
| Example 1 | 1.58 |
| Comparative Example 1 | >10000 |

Table 1 indicates that the compound of Example 1 has significantly high anti-HBV activity, as compared to the compound of Comparative Example 1.

### 2. HBs Antigen Level

Fig. 2 shows the measurement results of HBs antigen level. Fig. 2 indicates that as compared to known anti-hepatitis B virus agents (Entecavir and Tenofovir disoproxil fumarate), the HBs antigen level are remarkably reduced in the compound of Example 1.

### 3. Cytotoxicity

The CC₅₀ (50% cytotoxic concentration) was calculated from a graph showing the relationship between the concentration of the compound and cytotoxicity.

**Table 2**

| | CC₅₀ (µM) |
|---|---|
| Example 1 | 337.19 |

Table 2 indicates that the compound of Example 1 has low cytotoxicity.

### 4. SI value (Selectivity Index)

The SI value, which is an index of availability as a drug, was calculated by dividing the cytotoxic concentration (CC₅₀) by the effective concentration (EC₅₀). Specifically, SI=CC₅₀/EC₅₀.

**Table 3**

| | SI (CC₅₀/EC₅₀) |
|---|---|
| Example 1 | 213411 |

Table 3 indicates that the compound of Example 1 has a higher SI value (higher availability as a drug).

### Sequence Listing

P19-189WO_PCT_anti-hepatitis B virus agent
_20190927_132146_14.txt

## Claims

1. An anti-hepatitis B virus agent comprising, as an active ingredient, 2'-deoxy-2'-fluoro-β-2-fluoro-D-adenosine or its prodrug, or a pharmaceutically acceptable salt thereof, or a solvate thereof.

2. A prophylactic or therapeutic agent for a hepatitis B virus-related disease, comprising, as an active ingredient, 2'-deoxy-2'-fluoro-β-2-fluoro-D-adenosine or its prodrug, or a pharmaceutically acceptable salt thereof, or a solvate thereof.

3. The prophylactic or therapeutic agent according to claim 2, wherein the hepatitis B virus-related disease is one or more diseases selected from the group consisting of hepatitis B, type B liver cirrhosis, and type B liver cancer.


SEQUENCE LISTING

<110>  DAIKIN INDUSTRIES, LTD.
       KAGOSHIMA UNIVERSITY

<120>  Anti-Hepatitis B Virus Agent

<130>  P19-189WO

<150>  JP 2018-189216
<151>  2018-10-04

<150>  JP 2019-078616
<151>  2019-04-17

<160>  2

<170>  PatentIn version 3.5

<210>  1
<211>  18
<212>  DNA
<213>  Artificial

<220>
<223>  primer

<400>  1
gctcgtgtta caggcggg                                                    18


<210>  2
<211>  26
<212>  DNA
<213>  Artificial

<220>
<223>  primer

<400>  2
gaaccactga acaaatggca ctagta                                           26

SEQUENCE LISTING

<110>  DAIKIN INDUSTRIES, LTD.
       KAGOSHIMA UNIVERSITY

<120>  Anti-Hepatitis B Virus Agent

<130>  P19-189WO

<150>  JP 2018-189216
<151>  2018-10-04

<150>  JP 2019-078616
<151>  2019-04-17

<160>  2

<170>  PatentIn version 3.5

<210>  1
<211>  18
<212>  DNA
<213>  Artificial

<220>
<223>  primer

<400>  1
gctcgtgtta caggcggg                                                    18


<210>  2
<211>  26
<212>  DNA
<213>  Artificial

<220>
<223>  primer

<400>  2
gaaccactga acaaatggca ctagta                                           26